# EUROPÄISCHE PATENTSCHRIFT

(11) **EP 0 659 389 B1**
(45) Veröffentlichungstag und Bekanntmachung des Hinweises auf die Patenterteilung: **22.04.1998**
(21) Anmeldenummer: 93116918.9
(22) Anmeldetag: 20.10.1993
(51) Int. Cl.: A61F 2/06

(54) **Endoprothese**
Endoprothese
Endoprothèse

(43) Veröffentlichungstag der Anmeldung: 28.06.1995
(73) Patentinhaber: SCHNEIDER (EUROPE) AG, 8180 Bülach (CH)
(72) Erfinder: Gianotti, Marc, CH-8542 Wiesendangen (CH)
(74) Vertreter: EGLI-EUROPEAN PATENT ATTORNEYS

(56) Entgegenhaltungen:
- EP-A- 0 441 516
- EP-A- 0 556 940
- WO-A-91/12779
- US-A- 4 655 771
- US-A- 5 015 253

## Beschreibung

Die Erfindung betrifft eine Endoprothese gemäss dem Oberbegriff des Anspruchs 1 sowie ein Verfahren zu ihrer Herstellung. Derartige Endoprothesen (WO 91/12779) werden in der Medizin bei Gefahr von Gefässverschlüssen vor allem in Blutgefässen eingesetzt. Sie werden z. B. perkutan mittels eines Katheters an eine von einem Verschluss bedrohte Stelle einer Arterie gebracht und dort vorübergehend oder bleibend verankert, indem sie nach Abziehen einer Hülle elastisch expandieren oder durch eine Kraft von aussen geöffnet werden. Sie können jedoch auch in andere Gefässe wie z. B. Bronchien eingesetzt werden.

Derartige Endoprothesen sind z. B. aus der US-A-4 655 771 bekannt. Die dort beschriebenen Endoprothesen bestehen aus zwei miteinander verflochtenen, gegenläufigen Gruppen von gegeneinander verdrehten schraubenlinienförmig gebogenen Drähten aus elastischem rostfreiem Stahl. Solche Endoprothesen sind im Querschnitt stark komprimierbar und können daher gut eingeführt werden. Stahldrähte sind auch steif genug, um eine selbsttätige Expansion der Endoprothese nach der Einführung sicherzustellen. In der genannten Schrift ist auch die Möglichkeit erwähnt, Filamente aus Kunststoff oder Verbundmaterial zu verwenden, wobei jedoch der letztere Gedanke nicht weiter ausgeführt ist.

Aus EP-A-0 556 940 ist eine Endoprothese aus einem Schraubenfederartig gewundenen Metallkern mit einer PTFE Schicht und einer äußeren nicht-trombogenen Schicht bekannt.

Filamente aus Stahl haben wesentliche Nachteile. Sie sind nicht ideal biokompatibel und die Enden von Stahlfilamenten im Endoprothesengewebe müssen speziell behandelt werden, damit sie nicht Anlass zu Turbulenzen im Blutstrom geben und thrombogen wirken. Es ist daher nicht möglich, die Endoprothesen in einer Normlänge herzustellen und erst unmittelbar vor dem Einsatz auf die geeignete Länge abzuschneiden. Sie müssen in mehreren Längen hergestellt und gelagert werden.

Filamente aus Kunststoff sind zwar hinsichtlich ihrer Biokompatibilität günstiger, auch besser schneidbar - etwa mit Wasserstrahlschneidern, andererseits weisen sie oft nicht die erforderliche Steifigkeit auf oder sie müssen zur Erzielung derselben verhältnismässig dick sein, sodass die Endoprothese weniger komprimierbar und daher schwerer einzuführen ist als eine aus Stahldrähten aufgebaute.

Filamente aus Verbundmaterialien können die positiven Eigenschaften von Stahlfilamenten mit denen von Kunststoffilamenten verbinden. Dieser Vorteil allein war aber bisher noch nicht herausragend genug, um Filamenten aus Verbundmaterialien bei der Verwendung in Endoprothesen zum Durchbruch zu verhelfen.

Der Erfindung liegt die Aufgabe zu Grunde, eine Endoprothese der eingangs genannten Art anzugeben mit Filamenten aus Verbundmaterial, welche hohe Biokompatibilität und gute Schneidbarkeit mit günstigen mechanischen Eigenschaften, insbesondere einem kleinen Filamentdurchmesser und einer für die Erzeugung einer ausreichenden Federkraft genügenden Biegesteifigkeit der Filamente, vereinen, gleichzeitig sollen aber durch die neue Endoprothese der Ablauf einer Behandlung mit der Endoprothese in Bezug auf notwendige Handhabungen, auf Zuverlässigkeit und Sicherheit, auf Belastung des Patienten und in Bezug auf die Erfolgsaussichten verbessert werden. Ausserdem soll ein geeignetes Verfahren zur Herstellung einer derartigen Endoprothese angegeben werden.

Diese Aufgabe wird durch die Erfindung, wie sie in den Ansprüchen gekennzeichnet ist, gelöst.

Durch eine besondere Aufbereitung des Trägermaterials, die die Funktion des Trägermaterials während des Einsatzes der Endoprothese im menschlichen oder tierischen Körper über den Beitrag des Trägermaterials zur Biegesteifigkeit der Filamente hinaus erweitert, können von den beiden im Verbundmaterial zusammengefassten Materialien speziell dem Trägermaterial neue Aufgaben zugewiesen werden. Durch diesen durch die Erfindung neu zugänglich gemachten Funktionsträger kann die Endoprothese jetzt Aufgaben selbst übernehmen, die in Zusammenhang mit ihrem Einsatz zwar regelmässig anfallen, die vorher aber nur sehr umständlich, zeitaufwendig und für den Patienten belastend mit speziell darauf ausgerichten, von der Endoprothese unabhängigen Massnahmen zu erfüllen waren. Die Endoprothese kann jetzt bestimmte Funktionen automatisch und autark während ihres Einsatzes in dem Gefäss ausführen, sogar ohne spezielle Steuerung von aussen. Durch die Bereitstellung eines MehrzweckTrägermaterials wird die Behandlung mit der Endoprothese insgesamt effektiver, bei kleinerem Gesamtaufwand im Ergebnis wirkungsvoller. Die Belastung des Patienten mit Röntgenstrahlen, mit Röntgenkontrastmaterial oder mit Medikamenten und auch mit traumatischen Eingriffen kann deutlich reduziert werden. Durch den gezielten Einsatz der Massnahmen am Ort des Bedarfs werden die Erfolgsaussichten der Behandlung mit der Endoprothese grösser, sodass insgesamt der Einsatz von Verbundmaterialien für die Herstellung von Endoprothesen sich jetzt durchsetzen kann. Gleichzeitig kann trotz der zusätzlichen Aufgaben die hohe Biegesteifigkeit eines normalen Verbundfilaments leicht beibehalten werden. Das Trägermaterial muss im Verbund, anders als die Elemente hoher Zugfestigkeit, selbst keine Zugkräfte übertragen. Die Biegesteifigkeit wird im wesentlichen durch eine entsprechende Härte des Trägermaterials erzielt. Es reicht also zur Beibehaltung einer vorgebenen Biegesteifigkeit aus, wenn das Trägermaterial auch nach der besonderen Aufbereitung noch eine bestimmte Härte aufweist. Diese Bedingung ist leichter zu erfüllen als beispielsweise die Einhaltung einer bestimmten Zugfestigkeit bei werkstofftechnischen Veränderungen eines Ausgangsmaterials.

Die Filamente einer erfindungsgemässen Endoprothese können so geschnitten werden, dass sie an den Enden gerundet sind und keine Kanten aufweisen. Dadurch wird die Gefahr der Entstehung von thrombogen wirkenden Turbulenzen wesentlich entschärft. Sie können bei geringem Durchmesser ausreichend steif ausgebildet sein, sodass die Endoprothese sehr gut einführbar ist und trotzdem eine genügende Expansionskraft aufbringt.

Durch die Erfindung ist es möglich, beispielsweise die Oberfläche der Endoprothese sehr genau auf die jeweiligen medizinischen Anforderungen einzustellen, ohne dass die mechanischen Eigenschaften der Filamente dadurch wesentlich tangiert würden. Dies trifft in besonderem Masse zu, da durch die besondere Aufbereitung des Trägermaterials in der Filamentoberfläche des Trägermaterials eine das Filament umgebende, kalibrierte Hülle unlösbar verankert ist, die die Zugfestigkeit des Filaments nicht wesentlich verändert. Die Hülle kann sehr dünn sein, sodass sie die Komprimierbarkeit der Endoprothese nicht beeinträchtigt. Das Trägermaterial, dessen Härte die Steifigkeit des Filaments wesentlich bestimmt, kann dann ebenso wie die Elemente hoher Zugfestigkeit ohne Rücksicht auf die Biokompatibilität gewählt werden, während das Hüllenmaterial ganz auf die letztere Anforderung ausgerichtet sein kann. Das Trägermaterial ist in diesem Falle nicht nur Träger der Elemente hoher Zugfestigkeit, sondern auch noch Träger einer beispielsweise nach medizinischen Gesichtspunkten ausgewählten Hülle. Für diese Aufgabe kann eine Werkstoffpaarung Trägermaterial - Hülle ausgesucht werden, in der keines der beiden Paarungselemente der Anforderung nach hoher Zugfestigkeit genügen muss. Auch die Methode der Verankerung der Hülle am Trägermaterial kann jetzt ganz nach den Erfordernissen der Sicherheit ausgewählt werden, damit auf keinen Fall Hüllenmaterial sich vom Filament ablösen kann und beispielsweise in den Blutkreislauf des Patienten geraten kann. Es sind daher alle Möglichkeiten offen, um einer breiten Palette von möglichen Filamenthüllen mit jeweils unterschiedlichsten Funktionen eine nach medizinischen Gesichtspunkten sichere Basis zu verschaffen.

Eine besonders innige und damit sichere Verbindung des Hüllenmaterials mit dem Trägermaterial ergibt sich, wenn sowohl das Trägermaterial als auch das Material der Hülle jeweils einen Thermoplast enthalten, wenn der Thermoplast des Trägermaterials an der Filamentoberfläche angeschmolzen ist und mit dem Thermoplast des Hüllenmaterials verschmolzen ist. Das Trägermaterial kann dann besonders zuverlässig seine Aufgabe als Basis für medizinisch wirksame Filamenthüllen erfüllen. In besonderen Fällen, je nach medizinischer Aufgabe der Hülle und je nach Auswahl des Trägermaterials kann die Endoprothese noch weiter verbessert werden, wenn die Hülle den gleichen Thermoplast enthält wie das Trägermaterial.

Eine vorteilhafte Ausbildung ergibt sich auch, wenn das Träger- oder das Hüllenmaterial aus einem Gemisch von Stoffen aufbereitet ist, in dem ein Röntgenkontrastmittel enthalten ist. Die Lage der Endoprothese kann dann jederzeit, während der Einführung der Endoprothese und auch nach längerer Einsatzzeit röntgenologisch beobachtet werden, ohne dass dem Patienten dazu besonderes Röntgenkontrastmittel gegeben oder der Patient besonders hoher Röntgenstrahlungsbelastung ausgesetzt werden müsste. Das ist bei Einsatz der Endoprothese im Blutkreislauf besonders wichtig. Röntgenkontrastmittel kann zwar meistens mit dem Einführungskatheter der Endoprothese während der Einführung der Endoprothese nach Bedarf gegeben werden, nach einer längeren Einsatzzeit der Endoprothese müsste zur Röntgenkontrolle aber ein spezieller Katheter neu gesetzt werden, damit man das Kontrastmittel am Ort der Prothese in den Blutkreislauf bringen kann. Der traumatische Eingriff durch den neu gesetzten Katheter und die anderen Belastungen des Patienten werden durch die neue Entwicklung vermieden.

Wenn das an der Filamentaussenfläche liegende Material aus einem Gemisch von Stoffen aufbereitet ist, in dem ein im menschlichen oder tierischen Körper wirksames Medikament enthalten ist, dann bildet das Trägermaterial nicht nur ein die Biegesteifigkeit herbeiführendes Bett für die Elemente hoher Zugfestigkeit, sondern es bildet auch noch gleichzeitig eine implantierbare Medikamenten-Dosiervorrichtung. Das Medikament wird selbsttätig durch den Körper von der Endoprothese abgenommen, sobald diese im Körper eingesetzt ist. Eine speziell dosierte Medikamentengabe von aussen oder eine sonstige Steuerung von aussen ist nicht notwendig, der entsprechende Aufwand beim Arzt und beim Patienten entfällt. Dabei werden zwar zunächst vor allem Medikamente in Frage kommen, die in direktem Zusammenhang mit der Endoprothese stehen, die z.B. die Enstehung von Thromben an der Endoprothese verhindern sollen. Es sind jedoch auch noch andere Medikamente denkbar, vor allem ist denkbar, dass Endoprothesen entwickelt werden, die nur zum Zweck der Medikamentenabgabe in den menschlichen oder tierischen Körper eingesetzt werden. In jedem Fall erlaubt diese Endoprothese einen genau dosierten Einsatz der Medikamente und vor allem einen sehr gezielten Einsatz der Medikamente. Eine Belastung der restlichen Körperteile durch die Medikamente wird reduziert, die Gesamtmenge des Medikaments bleibt gering, am Ort des Bedarfs können aber hochwirksame Dosen eingesetzt werden.

Weiterhin vorteilhaft ist es, wenn durch die besondere Aufbereitung des Trägermaterials das an der Filamentaussenfläche liegende Material beim Übergang in seine feste Phase eine besonders grosse Oberfläche bildet, an der eine Beschichtung des Filaments verankert ist. Als Beschichtungen des Filaments kommen viele verschiedene Materialien und Techniken in Frage, beispielsweise ist eine Umhüllung der gesamten Endoprothese ein wichtiger Anwendungsfall, bei der beispielsweise eine Beschichtung der individuellen Filamente mit eingeschlossen ist. Als Beschichtung kommen auch biologisch abbaubare Schichten in Frage, die bei ihrem Abbau z.B. Medikamente abgeben. Bei Beschichtungen von Endoprothesen kommt es aber immer darauf an, dass die Beschichtung sich unter keinen Umständen unkontrolliert ablöst, damit im Körper keine wandernden Fremdkörper auftreten. Mit der grossen Oberfläche bietet das Trägermaterial eine gute Grundlage für eine zuverlässige Verankerung der verschiedenartigsten Beschichtungen. Nachbearbeitungen am Trägermaterial sind nicht notwendig, wenn die grosse Oberfläche bereits beim Übergang in die feste Phase des Trägermaterials gebildet wird.

Falls durch die besondere Aufbereitung des Trägermaterials das an der Filamentaussenfläche liegende Material Poren bildet, in die ein im menschlichen oder tierischen Körper wirksames Medikament eingelagert ist, entsteht eine besonders vorteilhafte Ausführung der Endoprothese. In dem nicht auf Zugfestigkeit ausgelegten Trägermaterial oder Hüllenmaterial können Poren leicht ohne Einschränkungen seiner Tauglichkeit als Träger- oder Hüllenmaterial vorgesehen werden. Eine poröse Aussenfläche bietet die besten Vorraussetzungen, festgelegte Mengen von flüssigen Stoffen aufzunehmen. Ausser der Fliessfähigkeit müssen die Stoffe dazu keine wesentlichen anderen Forderungen erfüllen, sodass ein weites Spektrum von Stoffen für diesen Zweck in Frage kommt. Die Menge des aufgenommenen Stoffes und die Abgabecharakteristik des Stoffes ist kontrollierbar durch die Tränkmethode, die Porengrösse und die Porenzahl, sodass diese Endoprothese besonders geignet ist für die Abgabe von Medikamenten der verschiedensten Dosierung.

Günstige Herstellverfahren für die Endoprothesen ergeben sich, wenn zur Herstellung eines Filaments auf Elemente hoher Zugfestigkeit Trägermaterial in Pulverform aufgebracht wird und dieselben dann durch eine geheizte Düse gezogen werden, und wenn weiterhin Hüllenmaterial in Pulverform auf das Filament aufgebracht und dasselbe dann durch eine weitere geheizte Düse gezogen wird. Dieses Herstellverfahren wird pultrudieren genannt und erfordert relativ wenig an Vorfertigung. Es lässt aber gleichzeitig Beimengungen in das in Pulverform vorliegende Trägermaterial zu.

Die Poren im Träger- oder Hüllenmaterial werden vorteilhafterweise hergestellt, indem dem an der Filamentaussenfläche liegenden Material vor dem Aufbringen auf die Elemente hoher Zugfestigkeit bzw. auf das bereits ausgeformte Filament eine lösliche körnige Substanz zugemischt und nach dem Durchziehen durch die beheizte Düse dieselbe durch ein Lösungsmittel aus der Filamentaussenfläche ausgewaschen wird.Auf diese Weise können mit sehr wenig Aufwand Poren gewonnen werden, die in ihrer Qualität und Verteilung besonders gut kontrollierbar sind.

Im folgenden wird die Erfindung anhand von lediglich Ausführungsbeispiele darstellenden Figuren näher erläutert.

Es zeigen
- Fig. 1a: eine erfindungsgemässe Endoprothese in expandiertem Zustand in Seitenansicht,
- Fig. 1b: die gleiche Endoprothese in expandiertem Zustand in Frontansicht,
- Fig. 2: die gleiche Endoprothese in komprimiertem Zustand in Seitenansicht,
- Fig. 3: einen Querschnitt durch ein Filament einer erfindungsgemässen Endoprothese gemäss einer ersten Ausführungsform,
- Fig. 4: einen Querschnitt durch ein Filament einer erfindungsgemässen Endoprothese gemäss einer zweiten Ausführungsform,
- Fig. 5: einen Querschnitt durch ein Filament einer erfindungsgemässen Endoprothese gemäss einer dritten Ausführungsform,

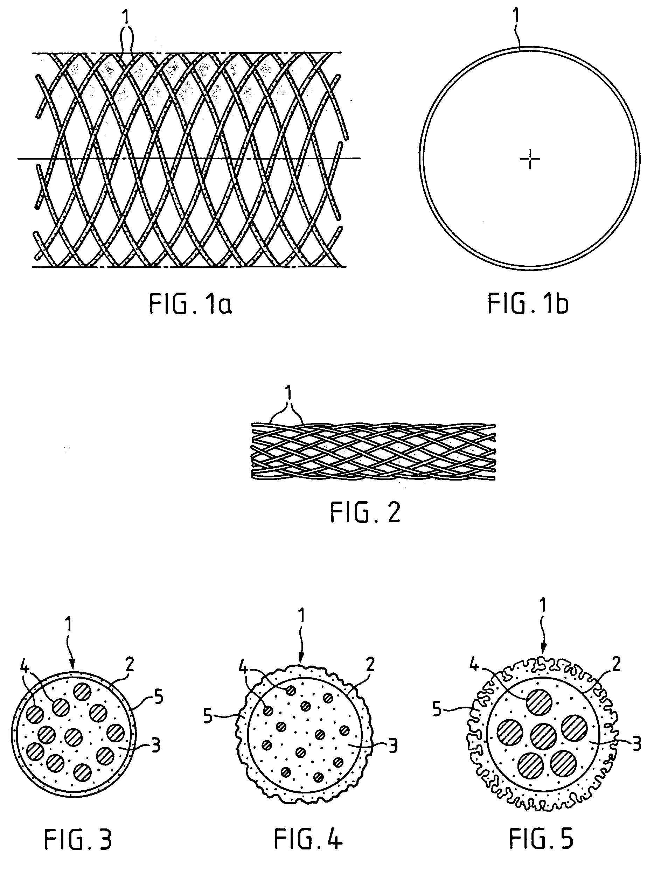

In Fig. 1a,b ist schematisch eine als rohrförmiges Geflecht ausgebildete Endoprothese dargestellt, welche aus einer Reihe biegesteifer elastischer und schraubenlinienförmig gebogener Filamente 1 besteht. Sie bilden zwei Gruppen entgegengesetzten Drehsinns von jeweils n Elementen, wobei die Filamente einer Gruppe über den Umfang gleich verteilt sind, sodass zwei benachbarte Filamente um 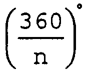 gegeneinander verdreht sind. n kann in weiten Grenzen schwanken, üblicherweise liegt es zwischen etwa 8 und 20. Die einander kreuzenden Filamente der beiden Gruppen sind miteinander verflochten, indem jedes Filament diejenigen der anderen Gruppe abwechselnd aussen und innen kreuzt. Dadurch erhält die Endoprothese eine feste, auch grösseren mechanischen Belastungen widerstehende Struktur.

Zugleich ist die Endoprothese jedoch gut komprimierbar. Unter dem Einfluss radial nach innen wirkender Kräfte wird sie, wie in Fig. 2 dargestellt, zusammengedrückt und gestreckt, wobei sich die Ganghöhe der Filamente erhöht, ohne dass sich die Struktur deswegen änderte. In dieser Form kann die Endoprothese z. B. in einer Hülle mittels eines Katheters verhältnismässig leicht eingeführt werden. Anschliessend kann die Hülle abgezogen werden, worauf die Endoprothese expandiert und sich im Gefäss zuverlässig verankert.

Wie in Fig. 3 - 5 dargestellt, besteht jedes Filament 1 aus einem Verbundmaterial 2, welches, in einem Trägermaterial 3 aus Kunststoff eingebettet, nebeneinander angeordnete, vorzugsweise etwa gleichmässig über die Querschnittsfläche des Verbundmaterials 2 verteilte, über die ganze Länge des Filaments 1 durchgehende Verstärkungsfasern 4 mit hoher Zugfestigkeit enthält. Die Steifigkeit des Filaments 1 kann durch die Härte des Trägermaterials 3 und durch Material und vor allem Dicke der Verstärkungsfasern 4 in weiten Grenzen nach Bedarf eingestellt werden. So ist - bei gleichen Materialien - das Filament nach Fig. 3 von mittlerer, dasjenige nach Fig. 4 von geringer und dasjenige nach Fig. 5 von hoher Steifigkeit. Auch durch die Führung der Verstärkungsfasern 4 - verdrillt oder parallel - können die mechanischen Eigenschaften des Filaments 1 gezielt beeinflusst werden. Die Durchmesser von Filamenten liegen im allgemeinen zwischen 0,05 und 0,25 mm. Dank dem geschilderten Aufbau sind schon bei geringen Durchmessern ausreichende Steifigkeiten erzielbar.

Als Verstärkungsfasern 4 kommen in erster Linie anorganische Fasern, insbesondere Kohle-, Kevlar- und Glasfasern in Frage. Kohlefasern haben den Vorteil guter Biokompatibilität.

Eine vollständige Ummantelung der Verstärkungsfasern 4 ist bereits durch das Trägermaterial 3 sichergestellt, welches vorzugsweise ein - möglichst hochfester - Thermoplast ist. Besonders geeignet sind Polyetheretherketon (PEEK) und Polyethersulfon (PS).

Das Filament 1 ist jeweils mit einer zusätzlichen Hülle 5 aus Kunststoff versehen. Sie kann - falls dasselbe biokompatibel ist, also etwa PEEK oder PS - im wesentlichen dem Trägermaterial 3 entsprechen, kann jedoch verschiedene Beimengungen und eine speziell behandelte Oberfläche aufweisen.

So weist das Filament nach Fig. 3 eine glatte Hülle 5 auf, während diejenige des Filaments nach Fig. 4 aufgerauht ist. Die Hülle 5 des Filaments nach Fig. 5 dagegen weist eine poröse Aussenschicht auf, welche sich besonders gut zur Aufnahme von Medikamenten oder anderen Wirkstoffen eignet, mit denen sie vor der Implantation getränkt wird und die sie nach der Implantation etwa an den Blutstrom oder umgebendes Gewebe abgibt. Die Geschwindigkeit der Abgabe lässt sich durch geeignete Wahl der Porengrösse einstellen. Es ist natürlich auch möglich, dem Hüllenmaterial Wirkstoffe direkt zuzumischen, welche dann sehr langsam freigegeben werden. Insbesondere kann ihm - oder auch dem Trägermaterial - ein Röntgenkontrastmittel wie Wismutzinkkarbonat beigemischt werden, sodass die Lage der Endoprothese im Körper leicht kontrolliert werden kann.

Die Poren können dadurch erzeugt werden, dass man dem Hüllenmaterial, bevor man es zur Herstellung der Hülle 5 auf das Verbundmaterial 2 aufbringt, lösliche körnige Substanz zumischt und nach Herstellung der Hülle 5 das Filament in ein geeignetes Lösungsmittel legt und die Körner auswäscht. Als körnige Substanz kann z. B. Salz, als Lösungsmittel Wasser verwendet werden.

Das Verbundmaterial 2 kann hergestellt werden, indem die Verstärkungsfasern 4 mit Trägermaterial in Pulverform bestreut und durch eine geheizte Düse gezogen - pultrudiert - werden, sodass das Trägermaterial zähflüssig wird und den Raum zwischen den Verstärkungsfasern 4 ausfüllt und dieselben umhüllt. Auf die gleiche Weise kann anschliessend die Hülle 5 aufgebracht werden, indem das Verbundmaterial 2 mit Hüllenmaterial in Pulverform - soll die Hülle 5 porös sein, kann es wie oben erläutert mit einer löslichen körnigen Substanz vermischt sein - bestreut und durch eine zweite geheizte Düse gezogen wird.

## Patentansprüche

1. Endoprothese zum Einsatz in Gefässe des menschlichen oder tierischen Körpers, mit einem rohrförmigen Geflecht aus biegesteifen elastischen Filamenten (1) welche aus einem Kernelement hoher Zugfestigkeit und aus einer das Kernelement umgebenden die Zugfestigkeit des Filamentes nicht wesentlich verändernden Hülle (5) zusammengesetzt ist, wobei die Hülle (5) zur Aufnahme von Wirkstoffen ausgebildet ist **dadurch gekennzeichnet**, **dass** das Kernelement aus einem Verbundmaterial (2) besteht, wobei das Verbundmaterial (2) aus mehreren Elementen (4) mit jeweils hoher Zugfestigkeit zusammengesetzt ist, die zur Erzielung einer hohen Biegesteifigkeit des Filaments von einem Trägermaterial (3) mit geringer Zugfestigkeit umhüllt sind, und die Hülle (5) auf dem Trägermaterial (3) unlösbar verankert ist.

2. Endoprothese nach Anspruch 1, **dadurch gekennzeichnet**, **dass** sowohl das Trägermaterial (3) als auch das Material der Hülle (5) jeweils einen Thermoplast enthalten, dass der Thermoplast des Trägermaterials (3) an der Filamentoberfläche angeschmolzen und mit dem Thermoplast des Hüllenmaterials zusammen verschmolzen ist.

3. Endoprothese nach Anspruch 2, **dadurch gekennzeichnet**, **dass** die Hülle (5) den gleichen Thermoplast enthält wie das Trägermaterial (3).

4. Endoprothese nach einem der Ansprüche 1 bis 3, **dadurch gekennzeichnet**, **dass** das Träger- oder das Hüllenmaterial (3,5) aus einem Gemisch von Stoffen aufbereitet ist, in dem ein Röntgenkontrastmittel enthalten ist.

5. Endoprothese nach einem der Ansprüche 1 bis 4, **dadurch gekennzeichnet**, **dass** das an der Filamentaussenfläche liegende Material aus einem Gemisch von Stoffen aufbereitet ist, in dem ein im menschlichen oder tierischen Körper wirksames Medikament enthalten ist.

6. Endoprothese nach einem der Ansprüche 1 bis 5, **dadurch gekennzeichnet**, **dass** das Trägermaterial (3) eine besonders grosse Oberfläche aufweist, an der die Hülle (5) verankert ist.

7. Endoprothese nach einem der Ansprüche 1 bis 6, **dadurch gekennzeichnet**, **dass** das an der Filamentaussenfläche liegende Material Poren aufweist, in die ein im menschlichen oder tierischen Körper wirksames Medikament eingelagert ist.

8. Verfahren zur Herstellung einer Endoprothese nach einem der Ansprüche 1 bis 7, **dadurch gekennzeichnet**, **dass** zur Herstellung eines Filaments (1) auf Elemente hoher Zugfestigkeit (4) Trägermaterial in Pulverform aufgebracht wird und dieselben dann durch eine geheizte Düse gezogen werden und dann Hüllenmaterial in Pulverform auf das bereits ausgeformte Filament (1) aufgebracht und dasselbe dann durch eine weitere geheizte Düse gezogen wird.

9. Verfahren nach Anspruch 8, **dadurch gekennzeichnet**, **dass** dem jeweils an der Filamentaussenfläche liegenden Material vor dem Aufbringen eine lösliche körnige Substanz zugemischt und nach dem Durchziehen durch die beheizte Düse dieselbe durch ein Lösungsmittel aus der Filamentaussenfläche ausgewaschen wird.

## Claims

1. Endoprosthesis for use in vessels of the human or animal body, with a tubular latticework made of flexurally rigid elastic filaments (1), which endoprosthesis is made up of a core element of high tensile strength and of a shell (5) which surrounds the core element and does not substantially alter the tensile strength of the filament, the shell (5) being designed to receive active substances, characterized in that the core element consists of a composite material (2), the composite material (2) being made up of a plurality of elements (4) in each case of high tensile strength, which, for the purpose of achieving a high flexural rigidity of the filament, are sheathed by a support material (3) of low tensile strength, and the shell (5) is anchored unreleasably on the support material (3).

2. Endoprosthesis according to Claim 1, characterized in that both the support material (3) and the material of the shell (5) each contain a thermoplastic, and in that the thermoplastic of the support material (3) is melted onto the filament surface and melted together with the thermoplastic of the shell material.

3. Endoprosthesis according to Claim 2, characterized in that the shell (5) contains the same thermoplastic as the support material (3).

4. Endoprosthesis according to one of Claims 1 to 3, characterized in that the support material or shell material (3, 5) is prepared from a mixture of substances which contains an X-ray contrast medium.

5. Endoprosthesis according to one of Claims 1 to 4, characterized in that the material lying on the filament exterior is prepared from a mixture of substances which contains a medicament effective in the human or animal body.

6. Endoprosthesis according to one of Claims 1 to 5, characterized in that the support material (3) has an especially large surface area, on which the shell (5) is anchored.

7. Endoprosthesis according to one of Claims 1 to 6, characterized in that the material lying on the filament exterior has pores in which a medicament effective in the human or animal body is incorporated.

8. Process for manufacturing an endoprosthesis according to one of Claims 1 to 7, characterized in that to manufacture a filament (1), support material in powder form is applied to elements of high tensile strength (4), and these are then drawn through a heated die, and then shell material in powder form is applied to the already shaped filament (1) and this is then drawn through a further heated die.

9. Process according to Claim 8, characterized in that, prior to the application, a soluble granular substance is admixed with the material lying in each case on the filament exterior, and after drawing through the heated die, this substance is washed out of the filament exterior by means of a solvent.

## Revendications

1. Endoprothèse à insérer dans des vaisseaux du corps humain ou animal, comprenant un treillis tubulaire constitué par des filaments élastiques (1) résistant à la flexion, qui sont composés par un élément de partie centrale manifestant une résistance élevée à la traction et par une enveloppe (5) entourant l'élément de partie centrale et qui ne modifie pas de manière essentielle la résistance à la traction manifestée par le filament, dans lequel l'enveloppe (5) est réalisée pour la réception de substances actives, caractérisée en ce que l'élément de partie centrale est constitué d'une matière composite (2), la matière composite (2) étant composée de plusieurs éléments (4) manifestant respectivement une résistance élevée à la traction, qui sont entourés, pour obtenir une résistance à la flexion élevée manifestée par le filament, d'une matière de support (3) manifestant une résistance minime à la traction, l'enveloppe (5) étant ancrée de manière inamovible sur la matière de support (3).

2. Endoprothèse selon la revendication 1, caractérisée en ce que aussi bien la matière de support (3) que la matière de l'enveloppe (5) contiennent respectivement une matière thermoplastique, en ce que la matière thermoplastique de la matière de support (3) est appliquée par fusion sur la surface des filaments et est portée à fusion de manière conjointe avec la matière thermoplastique de la matière d'enveloppe.

3. Endoprothèse selon la revendication 2, caractérisée en ce que l'enveloppe (5) contient la même matière thermoplastique que celle de la matière de support (3).

4. Endoprothèse selon l'une quelconque des revendications 1 à 3, caractérisée en ce que la matière de support ou la matière d'enveloppe (3, 5) est préparée à partir d'un mélange de substances qui contient un agent de contraste radiographique.

5. Endoprothèse selon l'une quelconque des revendications 1 à 4, caractérisée en ce que la matière appliquée sur la surface externe des filaments est préparée à partir d'un mélange de substances qui contient un médicament actif dans le corps humain ou dans le corps d'un animal.

6. Endoprothèse selon l'une quelconque des revendications 1 à 5, caractérisée en ce que la matière de support (3) présente une surface particulièrement grande à laquelle est ancrée l'enveloppe (5).

7. Endoprothèse selon l'une quelconque des revendications 1 à 6, caractérisée en ce que la matière appliquée sur la surface externe des filaments présente des pores dans lesquels est incorporé un médicament actif dans le corps humain ou dans le corps d'un animal.

8. Procédé pour la fabrication d'une endoprothèse selon l'une quelconque des revendications 1 à 7, caractérisé en ce que, pour la fabrication d'un filament (1), on applique une matière de support sous forme pulvérulente sur des éléments (4) manifestant une résistance à la traction élevée et on étire ensuite cette dernière à travers une filière chauffée, puis on applique une matière d'enveloppe sous forme pulvérulente sur le filament (1) déjà façonné et on étire alors ce dernier à travers une filière chauffée supplémentaire.

9. Procédé selon la revendication 8, caractérisé en ce qu'on ajoute, respectivement par mélange, à la matière disposée sur la surface externe des filaments, avant l'application, une substance granulaire soluble et, après étirage à travers la filière chauffée, on l'élimine de la surface externe des filaments par lavage à l'aide d'un solvant.
